# EUROPEAN PATENT APPLICATION

(11) **EP 1 384 790 A2**
(43) Date of publication of application: **28.01.2004**
(21) Application number: 03380136.6
(22) Date of filing: 05.06.2003
(51) Int. Cl.: C12Q 1/68

(54) **Labeling of objects to be identified consisting of at least one DNA fragment**

(30) Priority: 20.06.2002 AR 0202319
(71) Applicant: S.I.G. Sistemas de Identificacion Genetica, S.A., Cordoba (AR)
(72) Inventor: Simonetta, Rubén Antonio, Cordoba (AR); Jaime, Juan Carlos, Cordoba (AR); Sabagh, Julio César del Valle, Cordoba (AR)
(74) Representative: Urizar Anasagasti, José Antonio

(57) **Abstract**

A consistent marker is provided which contains at least one fragment of DNA, preferably a plurality of polymorphic DNA fragments of the type microsatellites (STR) and single nucleotide polymorphisms (SNP) microencapsulated and bound to a selected system of detection such as magnetic microspheres; pigments and a fluid with electrical properties, and/or fluorescent to ultraviolet and/or infrared radiation.

## Description

This invention consists in a marker of objects to be identified which includes at least one fragment of DNA; a procedure for the incorporation of the marker in the objects to be identified, and a method for the identification of the marked objects.

A concise description of the preferred method of execution will be provided in the following paragraphs in order to make this invention comprehensible so it can be easily put into practice. Such description is a demonstrative but not restrictive example of the invention whose components can be selected from among diverse equivalents without parting from the principles of the invention described herein.

LAYOUT - OBJECT: The search for elements, dispositions, and mechanisms to provide greater security to habitually conducted transactions is permanent. From the remote days when bills were created until today, man has looked for methods to prevent robberies, frauds, and forgeries. Perfecting printing methods , and incorporating security elements are clear examples of that search. Such methods have been surpassed the technical advances and the incipient quality of the equipment at the disposal of the general public. As an opposition to the erasure of checks to be written and cashed for higher amounts, the marking of paper begun to be used. The forgery of paper money and documents in general led to the adoption of special papers and inks, optical inks, the incorporations of security elements in the pulp, protection with translucent films, and so on. The inventors acknowledge the existence of elements such as fingerprints and DNA which are unique to each person. As a matter of fact, each individual possesses a particular biological trace which can be identified with almost absolute certainty by present-day technologies. The inventors also acknowledge that the incorporation of human DNA to an object for a positive and accurate identification could be easily exploited or used by forgers, since a simple sample of human DNA can be obtained just by being its possessor. A hair, saliva in a glass, a drop of blood, and even epithelial cells are enough to obtain the DNA necessary to be added to the counterfeit object, which would consequently seem to be the real one. According to this, the inventors have considered that the object to be identified will have to be marked with at least one specific fragment of DNA, and even better, with a combination of fragments of such DNA. Although forgers may be able to obtain the required DNA and incorporate it in the object, the will not know which fragment or fragments, among the million existing or their possible combinations, to use to achieve their aim. That is why, the aims of this invention are: to count on a marker of objects to be identified consisting of at least one fragment of DNA, to count on a procedure which allows for the incorporation of such markers in the objects to be identified, and to count on a method which allows for the identification of the objects marked with the marker.

### PRIOR ART:

Deoxyribonucleic acid (DNA) is a molecule of great size in which the genetic information of an individual is stored. Some sequences have specific functions, for example the genes and their regulators. Others, seemingly silent and whose functions are still ignored, are extremely abundant within the genoma.

Some sequences vary from one individual to another. For that reason, the comparison of these polymorphic regions constitutes the basis of molecular identification. The polymorphic regions are inherited from parents to children (mendelian inheritance), they therefore allow for the identification of a person by means of the comparison with individuals potentially related biologically.

The development of the techniques of DNA Typing, which allows the identification of any individual, has already entered into its adolescence. It has been fifteen years since the discovery that allowed the accurate identification of a person through the study of his or her DNA and the comparison of such DNA with the DNA of his or her relatives took place. Although the conceptual aspects underlying the process of identification have not substantially changed, the methodological aspects have experienced a vertiginous evolution.

The detection of new markers, the automation of some experimental stages, as well as the creation of computerized systems with great storage capacity and data analysis have marked a permanent trend in the field of human identification. Polymorphic sites are known in the human genoma from the year 1980. The ones which were first detected were those located in regions near some genes such as that of insulin. However, the detection of multiple variable regions (MLP) has made possible the development of systems of individual identification that were not restricted to the human species but which involved a great number of animal and vegetal species. This great contribution to scientific knowledge, with unsuspected application uses, was carried out by the English scientist Alec Jeffreys and his collaborators in the year 1985. Such variable regions scattered in all the genoma, allowed to show specific characteristics of individuals which are inherited in accordance with the mandelian inheritance. The impossibility of exactly locating the position of these regions in the genoma only allows to obtain a "molecular phenotype". Such phenotypic comparison makes the personal identification possible. Although this type of comparison is objective, its statistical evaluation, based on the population analysis, and its use allowed for the development of the first molecular system of human identification of great social impact, which was intensely used until the beginnings of the decade of the '90s. By the use of probes able to generate multilocus patterns to rake around in human libraries (the whole genetic information of an individual contained in microbial hosts), it was possible to detect variable sites previously located in the genoma and the markers of specific locus (SLP) able to define the genotype of an individual for a certain locus arose.

This method allowed for the replacement of its predecessor and contributed greatly with the standardization of the analytic procedures of human identification, particularly with those confronted by the FBI in the United States and by the Forensic Science Services of England. These robust genetic markers were used as the identification tool until the end of 1995. Later on, they were gradually replaced by the use of microsatellites or short tandem repeats (STR), which consist of polymorphic sequences of short repetition units and whose analysis depends on the amplification with the Polymerase Chain Reaction (PCR). These markers exhibit characteristics such as high sensibility which make them appropriate for forensic analysis, since minimum quantities of DNA are required for the analysis. It is due to this sensibility, to the speed of the analysis, and to the simple interpretation of the results that the microsatellites or STRs became the favorite forensic markers. Although at the moment the number of STRs is very high, a group of validated markers, which have been incorporated into standardized systems of analysis in commercial kits, has been selected. This group or kit of markers allows for the simultaneous analysis of up to 16 polymorphic human markers, thirteen of which constitute the basic group that has allowed the design of "intelligent databases."

The inventors acknowledge the fact that the Single Nucleotide Polymorphisms (SNP) appear approximately in one of every thousand pairs of bases, totaling in consequence more than three millions in the human genoma. In order to achieve a marking of objects which cannot be reproduced by forgers, the inventors proceed to incorporate in such objects polymorphic fragments of DNA, such as microsatellites (STR) and Single Nucleotide Polymorphisms (SNP). In this document, the inventors also reveal a procedure to incorporate these fragments to the objects to be identified as wll as a method which allows for the identification of the marked objects.

Document number CN1302905 refers to anticounterfeit material containing metallic ions of DNA prepared by mixing an aqueous solution of a soluble metallic salt with high coordination power with a solution of DNA and alcohol to obtain a decanted solution of soluble water M-DNA with gelatin, dextrin, the aqueous solution of soluble starch or rubber for the marking or ink to impress. Document number CN1306266 refers to a card of genetic identification, a method for the preparation of such card including the obtention of the owner's DNA, the processing of the information, and its impression.

Document number DE4446042 refers to a card that comprises an entity which provides a plurality of identifications of different species related chemically, for example by means of the use of a carrier of a material such as polystyrene, nitrocellulose, protein, polysaccharide or alcohol. The identifications can be enzymes, antibodies, antigens, and DNA. The cards can be used to authenticate bank notes, perfumes, documents, etc.

Document Number US6167518 refers to a digital certificate formed by a digital representation of a biological characteristic of the registrant; for example, the chromosomal DNA of the registrant. The representation has a personal message that is transmitted to the certificate itself. The identity of the registrant is verified in a remote way. The characteristics can be extracted from the certificate and be compared.

Document Number US6213391 refers to an identification system in which the identification starts from a distinctive biometric characteristic (for example, voice analysis, DNA, etc.). The biometric information is used in a variety of functions such as the control and security of transactions. An algorithm is provided for the creation of a key number to be used as a secondary identification code.

Document Number US6256737 consists of a system, method, and software that uses biometric measures for the authentication of users of entrepreneurial resources. A biometric control determines the method by which the user can be authenticated by the system. The execution includes the use of at least one biomedical parameter. It uses a scientific method for the identification of the user by comparison with unique characteristic, such as DNA. The method is disclosed to store the identification parameters.

Document Number US6312911 refers to a method to hide a message coded in a microdot using DNA and a method for the use of a mark with a coded message to identify objects.

Document number WO0068431 relates to a stenographic method to hide messages coded in DNA. The method comprises the uses of a sample of DNA hidden in a microdot and the labeling and authentication of objects of interest.

Document number WO0165375 relates to a system, method, and software that uses measuring to authenticate users of resources. It uses unique personal characteristics obtained by biometrics means and compared with those stored in a memory. The unique characteristics are, among others, the geometry of fingers and hands; analysis of facial and retinal image, voice, DNA, etc.

### DESCRIPTION

The present invention consists of a marker of objects to be identified which incorporates at least one fragment of DNA and, specifically, polymorphic fragments of DNA of the type of microsatellites (STR) and single nucleotide polymorphisms (SNP) to the objects to be identified.

The invention also consists of a procedure that includes: a first step, selection of a living being to proceed to the extraction of DNA from any one of its cells; a second step, purification of the obtained DNA; a third step, amplification of the polymorphic fragments of the type of microsatellites and single nucleotide polymorphisms; a fourth step, concentration and/or microencapsulation of the DNA; a fifth step, solubilization of the microcapsules of DNA; a sixth step, determination and/or correction of the degree of fluidity and concentration of the solution; and a seventh step, incorporation of the solution in an appropriate applicator and marking of the wanted object.

This invention also consists of a method for the identification of the marked object. In a first step, an appropriate detecting technique is used to detect a component incorporated to the solution and to individualize the marked object. In a second step, the key of the incorporated marker is obtained. In a third step, the authentication of the marked object is achieved by carrying out the necessary analysis.

### FUNCTIONING

Once the components of the invention and the sequence of stages developed to explain its nature are established, they are subsequently supplemented with their functional and operative relation and of the results provided.

In order to have a marker of objects to be identified that constitutes a secure means to protect valuables, this invention proposes the incorporation of a marker to these objects. Preferably, the inventors have considered that this marker will have to be a chemical compound that can be detected subsequently. The inventors prefer the use of deoxyribonucleic acid (DNA) as the marker of the objects to be identified. Particularizing, the inventors have considered that due to the great variety of combinations, it is rather impossible to reproduce the marking performed by means of the incorporation of polymorphic fragments of DNA of the type of microsatellites and single nucleotide polymorphism to the objects to be identified.

It is convenient to explain that when mention is made to DNA, the reference is in particular to the mentioned polymorphic segments of it. That is to say, those which make all living beings in the Earth different. Furthermore, when mention is made to individual polymorphic segments, it should be understood that such mention is only related to Short Tandem Repeats/Microsatellites (STR) and Single Nucleotide Polymorphism (SNP). At this stage, it is also necessary to mention the exception that only the univitelian twins will present polymorphic segments in common which will not allow their differentiation. However, with this invention and even in the event of univitelian twins, it will be possible to obtain a differentiating marking based on the combination of the individual polymorphic segments to be selected. As a matter of fact, when using a specific combination, the only person to know the exact site inside the thousands of polymorphic sites of the living being's genoma that has been used in the marking of the object is its owner. If necessary, and once this site is revealed, it will be possible to carry out a comparative analysis in any laboratory in the world that uses DNA Typing for the identification of people.

This circumstance is of supreme utility mainly in those cases in which it is necessary to obtain an identification of the object in other jurisdictions, and mainly, to appeal to justice.

In relation to the procedure for the incorporation of a consistent marker including at least one fragment of DNA in the objects to be identified, this invention comprises a plurality of steps.

In a first step, a living being is selected to later proceed to extract the DNA that will be used. The use of at least one fragment of a living being's DNA as a marker is proposed by the inventors. This should be interpreted in a wide sense, that is to say, whoever makes the decision of carrying out the labeling of their objects, will be able to select himself or herself as the donor of the DNA fragments or will be able to select any living being ( human, animal or vegetal being). As a consequence, the tiniest possibility of reproduction of the marker by forgers decreases even more. The extraction of the DNA starts from the extraction of cells or corporal fluids obtained by regular techniques, such as buccal swab; blood punction; the collection of epithelial cells, hair follicles, and the like.

In a second step, the obtained DNA is liberated in a solution compound by "Tris-CIH 10 mM" - EDTA 0.1 (mM), SDS to 20% (weight/volume) and Proteinase K 10 mg/ml. to proceed then to the purification with Phenol/Chloroform -10/9 (volume/volume).

In a third step, STRs and/or SNPs are amplified by using the Polymerase Chain Reaction (PCR) set forth in U.S. Patent Nos. 4,683,195; 4,683,202; and 4,800,159, which are incorporated by reference herein. The mixes that are placed into a thermocycler, contain the sample of DNA in a concentration of between 6 pgr. and 0.05 microgr., a PCR Buffer solution 10X, dNTP 10X, primers that flank the polymorphic region 10X of each one and Taq polymerase of 5000 units per ml.

In a fourth step, and in order to preserve the DNA from degradation, the procedure consists of concentration by ultracentrifugation for which microconcentrators such as Centricon 100 are used, and microencapsulating by means of the technique of phase inversion. In this step, the polymorphic DNA to be microencapsulated is dissolved in a solvent, and then, in the same solvent, a polymer is dissolved to a final concentration of between 0.25% and 10% weight/volume. The polymer used can be selected indistinctly between those biodegradable or those non-biodegradable. The preferred biodegradable ones are those such as lactic and glycolic acids and esters such as polyanhydrides, polyurethanes, butyric polyacid, valeric polyacid, and the like. Moreover, among the non biodegradable polymers vinyletylene acetate and acrylic polyacid are preferred. The use of polyamides and copolymers and of mixtures thereof is also accepted. The utilized polymers can also be selected from natural ones. In such a case, the use of dextran, cellulose, collagen, albumin, casein, and the like is preferred. The resulting mixture is introduced later on in a non-solvent in a relation solvent/non-solvent of at least 1/40 up to 4/200 to obtain the spontaneous formation of microcapsules. In this step, the solvent is an organic solvent selected among chloroform and methylene chloride, and the preferred non-solvents are ethanol and hexane.

Alternatively, in the fourth step, microcapsules with polycationics agents such as poly-L-lysine and ClNa are produced. In such a case,
- in a first stage, a polymer selected among those already mentioned is dissolved, among in an organic solvent such as chloroform.
- In a second stage of the fourth step, the polymorphic DNA is dissolved in water producing a first aqueous phase.
- In a third stage of the fourth step, the organic phase is emulsified with the first aqueous phase to obtain a first milky emulsion.
- In a fourth stage of the fourth step, the ClNa is dissolved in polyvinylic alcohol, producing a second aqueous phase.
- In a fifth stage of the fourth step, the first milky emulsion is emulsified with the second aqueous phase to produce a second milky emulsion.
- Lastly, in a sixth stage of the fourth step, the organic solvent of the second milky emulsion is evaporated, producing microcapsules containing polymorphic DNA.

Alternatively, in the fourth step, the DNA can be bound to magnetic microspheres or to pigments that are visible or not to the human eye or to a fluid or pigments with electrical properties and/or fluorescent to the ultraviolet and/or infrared radiation. Additionally, to mask the DNA fragment or the DNA fragments selected and to make the falsification of the marking even more difficult, a combination of the alternative techniques described in the fourth step can be used; and other fragments of DNA different from the chosen ones can be incorporated.

In a fifth step, the microspheres of DNA or the DNA microencapsulated are solubilized in a solution containing substances sensitive to ultraviolet radiation, such as fluorescein for example, tetrametil rhodamine, rhodamine 3, texas red, and the like, and/or substances sensitive to infrared radiation such as upconverted phosphor like gallium oxysulfur or lanthanides ions bound to a naphthalene group, and the like. The substances which are sensitive to the ultraviolet radiation and to the infrared radiation can be added free or microencapsulated with anyone of the techniques previously described. An alternative proposes to solubilize the microspheres of DNA or the microencapsulated DNA in a mixture of substances that are sensitive to infrared and ultraviolet radiation, for example, a mixture containing between 0.0001 and 0.02% ftalocianine in weight with a wavelength that oscillates between 670 and 720 nm and between 0.05 and 0.5% in weight of a selected fluorosphor among stilbene, dihydropyrazole, coumarin, carbostirilo and a compound of pirene with a wavelength that oscillates between 250 and 380 nm.

In a sixth step, the degree of fluidity and concentration of the solution that should be appropriate to facilitate its application in the objects to be marked, is determined and, if necessary, corrected. It has been considered that the degree of fluidity should allow for the applicator to deposit a solution with a concentration of between 6 pgr and 10 microgr. of marker per mm² surface.

In a seventh step, the marker is incorporated to an applicator that can be selected among a pen (pen having a nip, a ballpoint, or a felt tip), diverse types of filters, drawing instruments, paintbrush, stamp or some automatic machine such as inkjet printer and the like. Alternatively, an intermediary is used between the solution containing the marker and the object to be marked. In such a case, the intermediary is absorbed into the solution. The intermediary can be selected from among diverse substances, such as nitrocellulose, paper, wood, cardboard, plastic material, charged nylon, cloth, organic substances in form of drops or gel, inorganic substances, and the like.

Lastly, with the selected applicator, the designated objects are marked.

The method for the identification of the objects marked with at least one fragment of DNA includes a step of detection of the object by means of an appropriate system. To do so, the system can consist of a filter that facilitates the visualization of the pigments or the elements incorporated to the marker or a detector that allows for the verification of the presence of some kind of radiation, such as wavelength mixed with the sensitive substances, magnetic particles, or the characteristics of conductivity of the solution, and the like.

Diverse types of magnetic detection as well as the systems of electronic verification to detect a certain component according to their degree of conductivity are known in the prior art. In a first step, the object to be identified is detected. In a second step, the key of the incorporated marker is obtained. The third step consists in the authentication of the marked object to then carry out the analysis necessary for the typing of the polymorphic fragments of DNA by using the Polymerase Chain Reaction. The third step can only be carried out when the owner of the marked object reveals which is the oligonucleotides segment that has been placed on the object. Since this information is the starting point from which any forensic laboratory in the world that uses the technique of Polymerase Chain Reaction, as described, can proceed to amplify as needed such oligonucleotides segment and detect the polymorphic fragments of STR/SNP.

The detection of the fragments can be carried out by means of procedures and techniques that are usually used in the previous art. For instance, gels according to J. M. Robertson (1994); capillary electrophoresis according to McCord (1993); detection for multiple hibridization or multiple capillarity proposed by Y. Wang (1995), by the use of the microchips as stated by Woolley (1996); by Mass spectrometry according to Becker (1997); etc. In turn, the single nucleotide polymorphism can be detected by means of a conformational analysis of unique chain as demonstrated by Orita and others (1989); allelic oligonucleotide specific as Landeegren and others indicated (1988); multiple extension of primers according to Syvanen and others (1990) or other technologies such as chips, mass spectrometry, etc.

Likewise, in the event of a controversy, the rights of all the parties are guaranteed since it is possible to reproduce the test concerning the identity of the marked object as many times as necessary anywhere in the world since, as previously stated, the markers STR and SNP are routine procedures for the identification of people. On the other hand, it should be understood that these markers are recommended by the International Society for Forensic Genetics. With the marker described herein, it will be possible to identify with absolute certainty countless objects such as paintings, sculptures, sport inputs, works of art, handy crafts, videocassettes, recorders, televisions, home objects, computers, printers, software, office elements and business equipment. Likewise, it will be possible to identify perfumes, clothes, wallets, briefcases, boxes, parts of automobiles, airplanes, bicycles, paper money, checks, notarial documents, identification cards, driver's licenses, passports, visas, credit cards, telephone cards, and such similar objects as academic certificates, inventories, lottery tickets and other games of chance. In this way, one of the possible sequences of stages which lead to the concretion of this invention and the way it works has been described. The documentation is supplemented with the abstract of the invention within the claims that follow.

Once the nature of the invention, its scope, and the way it can be put into practice have been described, the following is declared as an invention and of exclusive property.

## Claims

1. A marker of objects to be identified that comprises at least one fragment of DNA of the type included in a given solution with a means for its detection, **characterized by** at least one fragment of DNA , preferably a plurality of polymorphic DNA fragments of the type microsatellites (STR) and single nucleotide polymorphisms (SNP) microencapsulated and bound to a selected system of detection such as magnetic microspheres; pigments and a fluid with electrical properties, and/or fluorescent to ultraviolet and/or infrared radiation.

2. A marker according to claim 1, **characterized by** the fact that at least one fragment of DNA is microencapsulated DNA and is incorporated in a solution containing at least one substance sensitive to ultraviolet radiation selected from the group consisting of fluorescein, tetrametil, rhodamina, rhodamina 3 and texas red, and mixtures thereof..

3. Marker according to claim 1, **characterized by** the fact that at least one fragment of DNA is microencapsulated and is incorporated in a solution containing substances sensitive to infrared radiation selected from, for example, an upconverting phosphor (giver) or lanthanide ions bound to a naphthalene group, among others.

4. Marker according to claim 1, **characterized by** the fact that at least one fragment of DNA is microencapsulated and is incorporated in a solution containing substances or pigments that are sensitive to ultraviolet radiation and/or infrared radiation.

5. Marker according to claim 1, **characterized by** the fact that at least one fragment of DNA is microencapsulated and is incorporated in a solution formed by a mixture of substances , sensitive to infrared radiation and ultraviolet radiation, containing between 0.0001 and 0.02% ftalocianine in weight with a wavelength of between 670 and 720 nm and between 0.05 and 0.5% in weight of a fluorosphor selected from the group consisting of stilbene, dihydropyrazole, coumarin, carbostirilo and compound of pirene and said fluorosphor has a wavelength of between 250 and 380 nm.

6. Procedure for the incorporation of the marker in the object to be identified, according to claim 1, comprising: (first step) extracting a sample containing DNA from a selected live being; (sixth step) determining and optionally correcting the degree of fluidity and concentration of the solution containing the extracted DNA, and (seventh step) incorporating the solution in an applicator; and marking the object to be identified. Procedure **characterized by** the fact that it comprises: (second step) liberating the obtained DNA in a solution compound with Tris-CIH 10mM - EDTA 0.1 mM, SDS to 20% (weight/volume) and Proteinase K 10mg/ml., purifying the DNA with Phenol/Chloroform 10/9 (volume/volume); (third step) amplification of microsatellites and single nucleotide polymorphism using polymerase chain reaction, obtaining a concentration of among 6 pgr. and 0.05 microgr. of the DNA sample, a solution PCR Buffer 10X, dNTP 10X, primers that flank the polymorphic region 10X of each one and Taq polimerase of 5000 units per ml., and placing of the solution and the DNA into a thermocycler; (fourth step) concentrating the DNA by ultracentrifugation; dissolving the solution containing DNA in a solvent and a polymer in a concentration of between 0.25 and 10% weight/volume, introducing a resulting mix in a non-solvent in a relation solvent/non-solvent of between 1/40 up to 4/200 and microencapsulating the DNA, and (fifth step) solubilizing the microcapsules of DNA in a solution containing substances sensitive to ultraviolet radiation and/or infrared radiation, selected from the group consisting of fluorescein, tetrametil, rhodamina, rhodamina 3 and texas red, and mixtures thereof, and / or substances sensitive to infrared radiation selected from, for example, an upconverting phosphor (giver) or lanthanide ions bound to a naphthalene group, among others.

7. A procedure according to claim 6, **characterized by** the fact that the fourth step involves producing the microcapsules containing polications agents selected from the group consisting of the poly - L - Lysine and ClNa, which involves: (first step) dissolving a polymer in an organic solvent, (second step) dissolving a polymorphic DNA in water producing a first aqueous phase; (third step) emulsifying the organic solvent with the first aqueous phase to obtain a first milky emulsion; (fourth step) dissolving the policationic agents in alcohol polyvinylic and water producing a second aqueous phase; (fifth step) emulsifying the first milky emulsion with the second aqueous phase to form a second milky emulsion, and (sixth step) evaporating the organic solvent of the second milky emulsion and producing microspheres containing polymorphic DNA

8. A procedure according to claim 6, **characterized by** the fact that I the fourth step the utilized polymer is biodegradable and is selected from the group consisting of lactic acid, glycolic acid, and an ester selected from the group consisting of polyanhydrides, polyurethanes, butiric polyacid and valeric polyacid, and mixtures thereof.

9. A procedure according to claim 6, **characterized by** the fact that in the fourth step the utilized polymer is non-biodegradable and is selected from the group consisting of vinyletilene acetate, acrylic polyacid, a polyamide, a copolymer, and mixtures thereof.

10. A procedure according to claim 6, **characterized by** the fact that in the fourth step the utilized polymer is a natural polymer selected from the group consisting of dextran, cellulose, collagen, albumin, and casein

11. A procedure according to claim 6, **characterized by** the fact that in the fourth step, the DNA is bound to magnetic microspheres or pigments that are visible or invisible to the human eye, to a fluid with electrical properties and/or fluorescent to ultraviolet and/or infrared radiation.

12. A procedure according to claim 6, **characterized by** the fact that in the fourth step, the organic solvent is selected from the group consisting of chloroform and methylene chloride, and the non-solvents are selected from the group consisting of ethanol and hexane.

13. A procedure according to claim 6, **characterized by** the fact that the fifth step involves adding a mix of substances sensitive to infrared radiation and ultraviolet radiation containing between 0.0001 and 0.02% in weight ftalocianine with a wavelength of between 670 and 720 nm and between 0.05 and 0.5% in weight of a fluorosphor selected from the group consisting of stilbene, dihydropyrazole, coumarin, carbostirilo and a compound of pirene with said selected fluorosphor having a wavelength of among 250 and 380 nm.

14. A procedure according to claim 6, **characterized by** the fact that the four step comprises adding microconcentrators of the type of the Centricon 100.

15. A procedure according to claim 6, **characterized by** the fact that the sixth step comprises providing a solution having a concentration of between 6 pgr and 10 microgr of marker per mm² of surface.

16. A procedure according to claim 6, **characterized by** the fact that the seventh step involves selecting the applicator from the group consisting of a pen having a nip, a ballpoint pen, a felt tip pen, a filter, a drawing instrument, a paintbrush, a stamp and automatic machines such as inkjet printers, and so on.

17. A procedure according to claim 6, **characterized by** the fact that the seventh step comprises incorporating into the solution containing the marker an intermediary selected from the group consisting of an organic substance in the form of drop or gel, and an inorganic substance.

18. A procedure according to claim 6, **characterized by** the fact that the seventh step comprises incorporating an intermediary selected from the group consisting of nitrocellulose, paper, wood, cardboard, plastic material, charged nylon and cloth.

19. Method for identifying the objects marked with the marker referred to in 1, obtained from the procedure described in claim 6, which involves (first step) detecting an object to be identified, (second step) obtaining identification of at least one DNA fragment in the marked object, and (third step) authenticating the marked object by detecting primers that flank a polymorphic region using Polymerase chain reaction.

20. Method according to claim 19, **characterized by** the fact that the first step (the detecting step) is carried out by means of a filter for visualizing at least one of pigments and elements incorporated into the marker, and by detecting at least one type of radiation such as the wavelength, magnetism, and/or conductivity incorporated with at least one radiation sensitive substance, magnetic particles, and the characteristics of conductivity of the solution.
